# EUROPEAN PATENT APPLICATION

(11) **EP 1 666 607 A1**
(43) Date of publication of application: **07.06.2006**
(21) Application number: 04747977.9
(22) Date of filing: 27.07.2004
(51) Int. Cl.: C12Q 1/68, C12Q 1/02, C12N 15/09, G01N 33/15, G01N 33/50, A61P 3/04

(54) **METHOD OF EVALUATING COMPOUND EFFICACIOUS IN TREATING OBESITY BY USING Slc25a10**

(30) Priority: 31.07.2003 JP 2003204249; 02.03.2004 JP 2004057535
(71) Applicant: BANYU PHARMACEUTICAL CO., LTD., Chuo-ku, Tokyo 103-8416 (JP)
(72) Inventor: KOTANI, Hidehito c/o Tsukuba Research Institute of, Tsukuba-shi, Ibaraki 3002611 (JP); ITADANI, Hiraku, c/o Tsukuba Research Institute of, Tsukuba-shi, Ibaraki 3002611 (JP); MIZUARAI, Shinji c/o Tsukuba Research Institute of, Tsukuba-shi, Ibaraki 3002611 (JP); ARAKI, Hiromitsu c/o Tsukuba Research Institute of, Tsukuba-shi, Ibaraki 3002611 (JP); MIKI, Satomi, c/o Tsukuba Research Institute of, Tsukuba-shi, Ibaraki 3002611 (JP)
(74) Representative: Rollins, Anthony John
(86) International application number: PCT/JP2004/010664
(87) International publication number: WO 2005/012570

(57) **Abstract**

Evaluation of compounds including screening of therapeutic agents for obesity is performed utilizing expression levels of S1c25a10 gene or protein in a test tissue or a test cell, or utilizing the nature of S1c25a10 gene or protein. Examination of obesity is performed based on expression levels of S1c25a10 gene or polymorphisms of the gene.

## Description

### Technical Field

The present invention relates to a method of evaluating compounds which are effective for treatment or prevention of obesity using S1c25a10 gene or protein. The invention further relates to an examination method for obesity using S1c25a10 gene or protein.

### Background Art

Obesity is a risk factor for numerous adult diseases including hypertension, diabetes, hyperlipidemia and ischemic heart disease. Since most of these are chronic conditions, they are expected to lead to rising medical costs and to create serious problems for society in the future.

Anti-obesity drugs are being developed for prevention, and currently several appetite suppressors and lipid absorption inhibitors are being used in the clinic. Some of the known target molecules in anti-obesity research include leptin, PPARγ and neuropeptide Y, but because of the huge variety of causes for obesity, it is desirable to focus on molecules having different action mechanisms as targets for future drug development.

Proper diagnosis of obesity and its causes is essential for appropriate treatment thereof, and therefore identification of a convenient and high-precision obesity marker has been desired. With the discovery in recent years that the effects of administered drugs are partially dependent on patient genotypes including genetic polymorphisms, it has become a goal to establish examination methods and diagnostic markers on the molecular level for clinical trials at the drug development stage, for so-called "tailor-made medicine".

S1c25a10 is known as a 6-membrane-spanning protein belonging to a group of transport proteins present in the mitochondrial inner membrane, and to date there have been published reports on cloning of rat and mouse genes (GenBank Accession No. NM_013770: SEQ ID NO: 1) (J. Biol. Chem. 273(38), 24754-24759 (1998): Non-patent document 1) and cloning of human gene (GenBank Accession No. NM_012140: SEQ ID NO: 2) (Biochem. J. 344, 953-960 (1999): Non-patent document 2).

Non-patent document 2 clearly demonstrates that S1c25a10 expression levels in mast cells are reduced when mice are exposed to cold, that S1c25a10 expression level are reduced when mouse 3T3-L1 cells are treated with insulin, and that S1c25a10 expression level are increased when the same cells are cultured in free fatty acids.

Non-patent document 1: Giuseppe Fiermonte, et al., J. Biol. Chem. 273(38), 24754-24759 (1998).
Non-patent document 2: Kallol Das, et al., Biochem. J. 344, 953-960 (1999).

### Disclosure of the Invention

### Problems to be Solved by the Invention

However, no knowledge has yet been published regarding the correlation between S1c25a10 and obesity or body weight, and no findings have been obtained about body weight-controlling compounds.

In light of the circumstances of the prior art as explained above, it is an object of the present invention to provide a method for evaluating compounds to allow screening of therapeutic agents for obesity. It is another object to provide an examination method for obesity which allows judgment to be made on the molecular level.

### Means for Solving the Problems

As a result of much diligent research directed toward achieving the aforestated objects, the present inventors discovered a fixed correlation between S1c25a10 gene expression levels and body weight, as well as a correlation between changes in S1c25a10 gene expression levels and changes in the expression of molecules connected with fatty acid synthesis, and thereupon completed the present invention.

Specifically, the present invention provides the following methods of evaluating compounds effective for treatment or prevention of obesity, (1) and (2).
(1) A method of evaluating compounds which are effective for treatment or prevention of obesity, characterized by comprising
   a step in which a test compound is administered to or contacted with a test animal or a test cell, and
   a step of detecting change in the expression level of S1c25a10 gene or a gene which is functionally equivalent to said gene, in said test animal or test cell.
(2) A method of evaluating compounds which are effective for treatment or prevention of obesity, characterized by comprising
   a step in which a test compound is administered to or contacted with a test animal or a test cell possessing a fusion gene comprising the expression regulatory region of S1c25a10 gene and a reporter gene, and
   a step of detecting changes in the expression level of the reporter gene in the test animal or test cells.

In the aforementioned evaluation methods (1) and (2), the change in expression level is preferably a reduction in the expression level. There may also be included a step of detecting change in at least one selected from ACC1 (acetyl-CoA carboxylase-1) expression, malonyl-CoA abundance and fatty acid abundance. Including such a step will allow judgment of whether the obesity of a subject is a result of S1c25a10-mediated fatty acid synthesis.

The present invention further provides the following methods of evaluating compounds effective for treatment or prevention of obesity, (3) and (4).
(3) A method of evaluating compounds which are effective for treatment or prevention of obesity, characterized by comprising a step in which a test compound is administered to or contacted with a test animal or a test cell, and a step in which it is confirmed whether or not the test compound exhibits an effect on the activity of S1c25a10 protein.
(4) A method of evaluating compounds which are effective for treatment or prevention of obesity, characterized by comprising a step in which a test compound is contacted with S1c25a10 protein, and a step in which it is confirmed whether or not the test compound exhibits an effect on the activity of the protein.

The invention still further provides therapeutic or preventing agents for obesity characterized by containing as active ingredients compounds obtained by any of the aforementioned evaluation methods.

The invention still further provides a method of inhibiting fatty acid synthesis characterized by lowering the expression level of S1c25a10 gene. The means for lowering the expression level of S1c25a10 gene is not particularly restricted, but preferably utilizes RNAi (RNA interference). The S1C25a10 RNAi may be accomplished, for example, by using the following siRNA (small interfering RNA): siRNA consisting of the nucleic acids of SEQ ID NOs: 3 and 4, siRNA consisting of the nucleic acids of SEQ ID NOs: 5 and 6, siRNA consisting of the nucleic acids of SEQ ID NOs: 7 and 8, siRNA consisting of the nucleic acids of SEQ ID NOs: 9 and 10, siRNA consisting of the nucleic acids of SEQ ID NOs: 11 and 12, siRNA consisting of the nucleic acids of SEQ ID NOs: 17 and 18, siRNA consisting of the nucleic acids of SEQ ID NOs: 21 and 22, siRNA consisting of the nucleic acids of SEQ ID NOs: 23 and 24, siRNA consisting of the nucleic acids of SEQ ID NOs: 25 and 26, siRNA consisting of the nucleic acids of SEQ ID NOs: 27 and 28, siRNA consisting of the nucleic acids of SEQ ID NOs: 29 and 30, siRNA consisting of the nucleic acids of SEQ ID NOs: 31 and 32, siRNA consisting of the nucleic acids of SEQ ID NOs: 35 and 36, siRNA consisting of the nucleic acids of SEQ ID NOs: 37 and 38, siRNA consisting of the nucleic acids of SEQ ID NOs: 39 and 40, siRNA consisting of the nucleic acids of SEQ ID NOs: 41 and 42, siRNA consisting of the nucleic acids of SEQ ID NOs: 43 and 44, siRNA consisting of the nucleic acids of SEQ ID NOs: 45 and 46, siRNA consisting of the nucleic acids of SEQ ID NOs: 47 and 48, siRNA consisting of the nucleic acids of SEQ ID NOs: 49 and 50. Particularly preferred are siRNA consisting of the nucleic acids of SEQ ID NOs: 9 and 10 and siRNA consisting of the nucleic acids of SEQ ID NOs: 41 and 42, because these siRNA significantly reduce expression of S1c25a10 gene.

The invention further provides a method for treating or preventing obesity, characterized by comprising a step of lowering the expression level of S1c25a10 gene. The means for lowering the expression level of S1c25a10 gene is not particularly restricted, but preferably is inhibition by RNAi. The RNAi is preferably accomplished using the siRNA mentioned above, and siRNA consisting of the nucleic acids of SEQ ID NOs: 9 and 10 and siRNA consisting of the nucleic acids of SEQ ID NOs: 41 and 42 are particularly preferred for use.

The invention still further provides the following obesity examination methods (1) to (5).
(1) A method of examining obesity characterized by assaying expression level and change in expression level of S1c25a10 gene in a test tissue or a test cell.
(2) A method of examining obesity characterized by assaying expression level and change in expression level of S1c25a10 protein in a test tissue or a test cell.
(3) A method of examining obesity characterized by assaying change in the amount of a substance involved in fatty acid synthesis resulting from change in expression level of S1c25a10 gene or activity of S1c25a10 protein in a test tissue or test cells.
(4) A method of examining obesity characterized by detecting a polymorphism in S1c25a10 gene in a test tissue or a test cell.
(5) A method of examining obesity characterized by detecting expression or activity of a protein which affects expression of S1c25a10 gene through interaction with S1c25a10 protein.

The invention still further provides siRNA characterized by being consisting of the nucleic acids of SEQ ID NOs: 9 and 10, as well as an S1c25a10 expression inhibiting agent, a fatty acid synthesis inhibiting agent and a therapeutic or preventing agent for obesity characterized by comprising the siRNA.

The invention still further provides siRNA characterized by being consisting of the nucleic acids of SEQ ID NOs: 41 and 42, as well as an S1c25a10 expression inhibiting agent, a fatty acid synthesis inhibiting agent and a therapeutic or preventing agent for obesity characterized by comprising the siRNA.

### Effect of the Invention

The evaluation method and examination method for compounds according to the invention provide a method for evaluating compounds to allow screening of therapeutic agents for obesity, while also providing an examination method for obesity which permits judgment to be made on the molecular level.

### Brief Description of the Drawings

Fig. 1 is a micrograph of HEK293 cells, wherein (a) shows CCCP-treated HEK293 cells and (b) shows CCCP-non-treated HEK293 cells.
Fig. 2 shows the results of flow cytometry analysis showing the relationship between S1c25a10 and mitochondrial proton gradient, wherein (a) represents CCCP-treated HEK293 cells, (b) represents non-treated HEK293 cells and (c) represents S1c25a10-transferceted HEK293 cells.
Fig. 3 is a bar graph showing the results of fluorometric analysis of the relationship between S1c25a10 and mitochondrial proton gradient.
Fig. 4 is a photograph showing S1c25a10 gene expression levels in different tissues.
Fig. 5 is a pair of graphs showing changes in expression levels of the (a) mouse S1c25a10 gene and (b) ACC1 gene before and after differentiation to adipocytes.
Fig. 6 is a pair of graphs showing relative expression levels of S1c25a10 gene in various siRNA-transfected cells, wherein (a) represents the results for mouse cells and (b) represents the results for human cells. SCR stands for cells transfected with scramble siRNA which produces no RNAi in mammals.
Fig. 7 is a pair of graphs showing expression levels of the (a) S1c25a10 gene and (b) ACC1 gene in cells transfected with siRNA (H4 or H10).
Fig. 8 is a graph showing expression levels of the ACC1 gene in cells overexpressing S1c25a10 gene.
Fig. 9 is a graph showing malonyl-CoA levels in cells transfected with siRNA (M8).
Fig. 10 is a set of micrographs of 3T3-L1 cells differentiated to adipocytes, wherein (a) shows the cells before differentiation, (b) shows the cells after differentiation, (c) shows the differentiated cells after treatment with scr-siRNA and (d) shows the differentiated cells after treatment with siRNA (M8).
Fig. 11 is a bar graph showing amounts of fat accumulation in each of the cell types.

### Best Mode for Carrying Out the Invention

Preferred modes of the invention will now be explained in detail.

The terms used for description of the present invention will now be explained.

"Expression level" according to the invention refers to the absolute or relative amount of transcription product of S1c25a10 gene. The term "gene" includes both DNA and mRNA. When the target of expression detection is the protein, the "expression level" refers to the absolute or relative amount of translation product of S1c25a10 gene.

A "test animal" according to the invention is not particularly restricted in terms of species, and as examples there may be mentioned mouse, rat, rabbit, dog, monkey and the like.

The type of "test tissue" according to the invention is not particularly restricted so long as it is a tissue which can be extracted from the body for examination of obesity, but from the standpoint of readily reflecting effects on obesity it is preferably liver tissue, adipose tissue, muscle tissue or blood tissue. From the standpoint of facilitating isolation of the tissue, it is most preferably blood tissue. There are no particular restrictions on the animal species from which the tissue is taken, but human tissue is preferred since the major purpose of the invention will be for human clinical use.

The type of "test cell" according to the invention is also not particularly restricted so long as the cell can be extracted from the body for examination of obesity, but from the standpoint of readily reflecting effects on obesity it is preferably hepatocyte, adipocyte (white adipocyte, brown adipocyte, etc.), muscle cell (myoblast, skeletal muscle cell, smooth muscle cell, etc.), pancreatic cell (islet cell, etc.) or hemocyte. There are no particular restrictions on the animal species from which the cells are derived, but human cells are preferred since the major purpose of the invention will be for human clinical use.

"Obesity" according to the invention includes not only general obesity as defined by an excess accumulation of adipose tissue, but also "adiposity" associated with complications such as diabetes or hypertension, or visceral fat. "Obesity" according to the invention may also refer to a state of increased body weight relative to an original body weight, in the case of body weight control by administration of a drug or the like.

The term "examination" used according to the invention includes not only simple discernment of obesity but also "prognosis" regarding future obesity.

S1c25a10 according to the invention will now be described. S1c25a10 is found in the mitochondria of brown adipocytes, and it has approximately 36-38% homology with the UCP family of proteins associated with thermogenesis. UCP protein causes heat production in the mitochondria. Specifically, protons produced by oxidation of glucose and fatty acids are released from the mitochondrial inner membrane to the outside of the membrane by respiratory enzymes, resulting in creation of a proton gradient. UCP functions as a channel to transport protons into the inner membrane, thus reducing the proton gradient. As a result, oxidation of fatty acids, etc. by protons in inner membrane is accelerated, thereby promoting thermogenesis. In other words, UCP has the function of promoting energy consumption in the body.

The present inventors have found that, despite the high homology between S1c25a10 and UCP mentioned above, S1c25a10 has the opposite activity to UCP on thermogenesis in mitochondria. That is, S1c25a10 is present in mitochondrial inner membrane and has activity which increases a proton gradient between the inner and outer mitochondrial membranes, such that it functions to accumulate energy without thermogenesis. Thus, the present inventors consider that inhibiting the function of S1c25a10 should promote energy consumption and contribute to anti-obesity.

The present inventors also discovered that a correlation exists between changes in expression levels of S1c25a10 gene and changes in expression levels of molecules associated with fatty acid synthesis. Specifically, when S1c25a10 gene expression are inhibited, expression levels of ACC1 (acetyl-CoA carboxylase), a gene involved in fatty acid synthesis, and levels of malonyl-CoA, a fatty acid precursor, are reduced, while on the other hand they increase when S1c25a10 gene expression is increased. This substantiates the hypothesis that S1c25a10 is a molecule which either lies in the fatty acid synthesis pathway or is intricately involved in the synthesis pathway. Fatty acids are constituents of lipids and free fatty acids in adipose tissue are converted to triacyl glycerols and stored in adipocytes. In other words, inhibiting synthesis of fatty acids can prevent storage of triacyl glycerols in adipocytes. Since fatty acid synthesis can be reduced by lowering the expression level of S1c25a10 or of genes or molecules involved in the synthesis pathway, the behavior (expression, activity, etc.) of such genes or molecules can be used as an index for evaluation or selection of compounds which are effective for obesity.

### (1) Method of evaluating compounds effective for treatment or prevention of obesity

A method of evaluating compounds which are effective for treatment or prevention of obesity will now be explained. If a test compound is administered to or contacted with a test animal or a test cell and the resulting variation in S1c25a10 gene expression is assayed, or the test compound is contacted with S1c25a10 protein and the effect on the protein activity is examined, it is possible to evaluate the test compound.

Specifically, it is thought that such test compounds will include those which act on cells or tissues to normalize or control S1c25a10 gene expression levels or S1c25a10 protein activity, thereby helping to normalize mechanisms that contribute to obesity, e.g. controlling fat accumulation and appetite. Thus, the evaluation method described below allows evaluation of compounds which are effective for treatment or prevention of obesity.

### (A) Evaluation method using S1c25a10 gene expression level regulation as index

As a first evaluation method using S1c25a10 gene expression level regulation as an index, there may be mentioned a method in which a test compound is administered to or contacted with a test animal or a test cell and it is confirmed whether or not the test compound regulates expression levels of S1c25a10 gene or a gene which is functionally equivalent to the gene, in the test animal or test cell. This method allows identification of compounds which are effective for treatment or prevention of obesity.

Specifically, a test compound is evaluated by the following procedure. First, the test compound is administered to or contacted with the test animal or test cell. There are no restrictions on the type of test compound, regardless of its structure or properties, so long as it is a candidate compound for treatment or prevention of obesity. The mode of administering the test compound to a test animal is not particularly restricted, and specifically there may be mentioned, for example, oral administration and parenteral administration (such as percutaneous administration, intramuscular injection, intravenous injection or subcutaneous injection). There are also no particular restrictions on the method of contacting the test compound with test cell, and specifically there may be mentioned, for example, methods of contact by admixture in a solution such as a culture solution or buffer solution (phosphate buffer or the like).

It is then confirmed whether or not the test compound regulates the level of expression of S1c25a10 gene or a gene which is functionally equivalent to that gene in test animals or test cells.

There are no particular restrictions on the method of confirming whether or not the expression level of the gene is regulated, and it may be carried out by detecting and comparing change in the gene expression level by a gene amplification method such as RT-PCR, a method using a DNA microarray or a Northern hybridization method, against the pre-administration or pre-contact levels as a control. There may optionally be used animals or cells having artificially introduced therein a fused gene comprising the expression regulatory region of the gene and a reporter gene. For such cases, specific examples of reporter genes include β-galactosidase gene, luciferase gene and green fluorescence protein gene.

Here, "a gene which is functionally equivalent to S1c25a10 gene" refers to a gene which has a different nucleotide sequence than S1c25a10 gene but exhibits relatively high homology and has identical or similar activity to S1c25a10. The degree of homology is not particularly restricted so long as the functions of the genes are equivalent, but the nucleotide sequence homology is preferably 70-100%, more preferably 80-100% and even more preferably 90-100%. If the homology is lower than this range, the gene is probably one which does not exhibit identical or similar function to S1c25a10. However, even if the nucleotide sequence homology is below the aforementioned range, the gene may still have identical or similar function to S1c25a10 gene if there is high homology between the domain exhibiting the unique function of S1c25a10 and the nucleotide sequence corresponding to that domain. Such genes can be suitably used even if the nucleotide sequence homology falls outside of the aforementioned range. In addition, a gene with relatively high homology can be obtained by natural or artificial substitution, deletion, addition and/or insertion of one or more bases of S1c25a10 gene.

When the expression level of S1c25a10 gene or the gene which is functionally equivalent to S1c25a10 gene is reduced by at least 30% and preferably at least 50% after administration of or contact with the test compound compared to the level before administration of or contact with the test compound, the test compound may be evaluated as a compound effective for treatment or prevention of obesity.

As a second evaluation method using S1c25a10 gene expression level regulation as an index, there may be mentioned a method in which a test compound is administered to or contacted with a test animal or a test cell and change in the expression level of S1c25a10 gene or a gene which is functionally equivalent to the gene in the test animal or test cell is detected. By detecting not only regulation of the expression level but also the degree of change in the expression level, it is possible to perform an evaluation which also takes into account the strength of activity of the test compound. Also, as mentioned above, reduction in the expression level of S1c25a10 gene reduces expression and abundance of molecules in the fatty acid synthesis pathway such as ACC1 and malonyl-CoA, thus effectively lowering synthesis of fatty acids. For screening of compounds that lower fatty acid synthesis ability, therefore, the index employed in this second evaluation method is preferably reduction in expression levels of S1c25a10 gene or a gene which is functionally equivalent to the gene.

There are no particular restrictions on the method of detecting changes in the expression level of the gene, and it may be carried out by detecting and quantitating change in the gene expression level by a gene amplification method such as RT-PCR, a method using a DNA microarray or a Northern hybridization method, against the pre-administration or pre-contact levels as a control. There may optionally be used animals or cells having artificially introduced therein a fused gene comprising the expression regulating region of the gene and a reporter gene. For such cases, specific examples of reporter genes include β-galactosidase gene, luciferase gene and green fluorescence protein gene.

When the expression level of S1c25a10 gene or the gene which is functionally equivalent to S1c25a10 gene is reduced by at least 30% and preferably at least 50% after administration of or contact with the test compound compared to the level before administration of or contact with the test compound, the test compound may be evaluated as a compound effective for treatment or prevention of obesity.

The aforementioned second method may also include a step of detecting change in at least one selected from ACC1 expression levels, malonyl-CoA abundance and fatty acid abundance, which accompany changes in S1c25a10 gene expression levels. Here, ACC1 refers to either ACC1 protein or gene.

The method of detecting changes in the expression level of ACC1 protein or gene is not particularly restricted, and for example, there may be mentioned Western blotting, Southern hybridization, Northern hybridization, a DNA chip and RT-PCR. The method of detecting changes in malonyl-CoA abundance is also not particularly restricted, and for example, there may be mentioned a method in which cell-derived malonyl-CoA partially purified by reverse phase chromatography or the like is reacted with a fatty acid synthase and radioactively labeled acetyl-CoA, and the labeled fatty acid which is produced is measured.

When the expression level of ACC1 gene or ACC1 protein or the malonyl-CoA or fatty acid abundance is reduced by at least 5% and preferably at least 10% after administration of or contact with the test compound compared to the level before administration of or contact with the test compound, the test compound may be evaluated as a compound effective for treatment or prevention of obesity.

Thus, evaluation of compounds based on S1c25a10 gene expression levels and ACC1, malonyl-CoA and fatty acid expression levels or abundance allows evaluation of test compounds which directly act on the fatty acid synthesis pathway.

### (B) Evaluation method using S1c25a10 protein activity as index

If a test compound is contacted with a test animal, test cell or S1c25a10 protein and it is confirmed whether or not the test compound affects activity of the protein, it is also possible to evaluate test compounds which are effective for treatment or prevention of obesity.

Specifically, a test compound may be evaluated by the following procedure. First, a test compound is contacted with a test animal, test cell or S1c25a10 protein. There are no restrictions on the type of test compound, regardless of its structure or properties, so long as it is a candidate compound for treatment or prevention of obesity. The mode of administering the test compound to a test animal is not particularly restricted, and specifically there may be mentioned, for example, oral administration and parenteral administration (such as percutaneous administration, intramuscular injection, intravenous injection or subcutaneous injection). There are also no particular restrictions on the method of contacting the test compound with a test cell, and specifically there may be mentioned, for example, methods of contact by admixture in a solution such as a culture solution or buffer solution (phosphate buffer or the like). There are likewise no particular restrictions on the method of contacting the protein and the test compound, and specifically there may be mentioned methods of contact by admixture in a solution such as a buffer solution (phosphate buffer or the like).

It is then confirmed whether or not the test compound affects the activity of the protein. The conditions for assaying the protein activity may be appropriately set depending on the nature of the protein used. The specific conditions, in the case of S1c25a10 protein for example, may be based on changes in the proton gradient between inside and outside of mitochondrial inner membrane (for example, see Yu XX et al., Biochem. J (2001) 353, 369-375).

When the activity of the S1c25a10 protein is reduced by at least 30% and preferably at least 50% after administration of or contact with the test compound compared to the level before administration of or contact with the test compound, the test compound may be evaluated as a compound effective for treatment or prevention of obesity.

The method of evaluating compounds effective for treatment or prevention of obesity according to the invention as explained above allows screening of therapeutic or diagnostic agents for obesity, evaluation of the efficacy and safety of such agents, and selection of appropriate agents for tailor-made therapy.

### (2) A method of examining obesity

A method of examining obesity according to the invention will now be explained.

### (A) A method of examining obesity based on assay of S1c25a10 gene expression levels

By detecting changes in the expression level of S1c25a10 gene or assaying its expression level in a test tissue or a test cell, it is possible to perform examination or diagnosis regarding obesity of the organism (for example, a human) from which the test tissue or test cell have been extracted. This allows not only examination of the condition of obesity at the time of examination, but also permits prognosis regarding possible future obesity.

A specific method for such examination will now be explained. First, the test tissue or test cells are extracted from an organism as the subject of examination. There are no particular restrictions on the method of extraction, and any publicly known method may be employed.

Next, the gene whose expression level is to be assayed is prepared from extracted test tissue or test cell. Measurement of S1c25a10 gene expression level requires preparation of S1c25a10 RNA (total RNA or mRNA) from the test tissue or test cell. The RNA can be prepared by a publicly known method, with reference to, for example, Molecular cloning A LABORATORY MANUAL 2nd EDITION (1989) (T. Maniatis: Cold Spring Harbor Laboratory Press) 7.3-7.36. The prepared RNA may then be used for measurement of the expression level by, for example, a gene amplification method such as RT-PCR, a method using a DNA microarray (for example, an Affymetrix DNA chip) or a Northern hybridization method. The expression level may also be measured by *in situ* hybridization or the like, using the test tissue or test cells.

For detection of changes in the expression level of S1c25a10 gene, the change in expression level may be determined by assaying the expression level before and after a period in which the expression level is expected to change (for example, before and after administration of an obesity therapeutic agent). Specifically, it is possible to determine that an increase in body weight has occurred or may occur in the future if expression level of S1c25a10 gene in a test tissue or test cell is significantly increased before and after a period in which the expression level is expected to change. If the expression level is reduced, on the other hand, it may be determined that a decrease in body weight has occurred or may occur in the future.

### (B) A method of examining obesity based on assay of S1c25a10 protein expression levels

By detecting change in expression level of S1c25a10 protein in a test tissue or a test cell, or by assaying the expression level, it is possible to perform examination or diagnosis regarding obesity of the organism (for example, human) from which the test tissue or test cell has been extracted. This allows not only examination of the condition of obesity at the time of examination, but also permits prognosis regarding possible future obesity or emaciation.

A specific method for examination will now be explained. The method for protein expression level assay may be a method of quantitating protein isolated from an organism or a method of assaying protein levels in the blood, and there are no particular restrictions on the actual method employed. A specific method for quantitation of protein isolated from an organism is described below. First, S1c25a10 protein is prepared from a test tissue or a test cell. The protein preparation may be carried out by a publicly known method. The expression level can be measured from the prepared protein using a method employing a protein chip (for example, Protein Chip System by CIPHERGEN) or an immunological method (for example, ELISA, EIA or Western blotting). The expression level can also be measured by immunostaining of the test tissue or test cell. As a specific example of a method of measuring protein levels in the blood there may be mentioned quantitation of S1c25a10 protein by an immunological method as mentioned above, using sampled blood from the organism.

Thus, by analyzing the results after assaying S1c25a10 gene or protein expression levels in the manner described above, it is possible to examine the state of obesity or emaciation of a subject. That is, according to the present invention, a fixed correlation between S1c25a10 protein expression level and body weight has been established, and therefore comparison of the examination results with the S1c25a10 protein expression level of a control group (healthy individuals) allows judgment of the severity of obesity. The examination method of the invention allows not only examination of the state of obesity at the time of examination, but also permits prognosis regarding possible future obesity or emaciation.

For detection of change in the level of expression of S1c25a10 protein, the change in expression level may be determined by measuring the expression level before and after a period in which the expression level is expected to change (for example, before and after administration of an obesity therapeutic agent). Specifically, it is possible to determine that an increase in body weight has occurred or may occur in the future if expression level of S1c25a10 protein in a test tissue or test cell is significantly increased before and after a period in which the expression level is expected to change.

### (C) A method of examining obesity based on interaction between S1c25a10 and molecules involved in the fatty acid synthesis pathway

S1c25a10 gene and protein exhibit their characteristic function by direct or indirect interaction with many other molecules. For example, the present inventors have found a correlation between S1c25a10 gene expression levels and expression levels of various molecules in the fatty acid synthesis pathway (for example, ACC1 and malonyl-CoA), and have discovered that increased expression of S1c25a10 gene leads to increased expression of molecules in fatty acid synthesis pathway while decreased expression of S1c25a10 gene leads to decreased expression of molecules in the fatty acid synthesis pathway. This means that measuring expression levels of S1c25a10 gene or protein allows detection of the activated state of the fatty acid synthesis pathway. As a result, it is possible to predict and examine fat accumulation due to accelerated fatty acid synthesis.

The method of detecting expression of such molecules in fatty acid synthesis pathway is not particularly restricted and may be detection by Northern hybridization or RT-PCR in the case of a gene (for example, ACC1), or in the case of malonyl-CoA, the method may involve fatty acid synthesis reaction using malonyl-CoA as the substrate, and calculation of the amount of malonyl-CoA based on incorporation of a radioactive isotope.

Changes in levels of molecules in fatty acid synthesis pathway detected in the manner described above may compared with the expression level or amount of the molecule in healthy persons or persons with standard body weight, and a significant increase in the change will allow diagnosis regarding increased body weight or a possible future increase in body weight.

By detecting changes in the levels of molecules in fatty acid synthesis pathway resulting from changes not only in the expression of S1c25a10 but also in the activity of S1c25a10 protein, it is possible to perform examination of obesity.

### (D) A method of examining obesity by detection of gene polymorphisms in S1c25a10 gene

When gene polymorphisms are present in S1c25a10 gene, expression levels of S1c25a10 gene or protein vary depending on the existence and types of such polymorphisms, and can often abnormally affect activity of the protein. Thus, detection of such gene polymorphisms can yield knowledge regarding S1c25a10 expression and activity, while also allowing examination regarding obesity of a subject from which a test tissue or test cell is derived. Such polymorphisms include, specifically, minisatellites, microsatellites and SNPs (single nucleotide polymorphisms).

Detection of polymorphisms in S1c25a10 gene may be accomplished in the following manner. Specifically, the base sequence of a region which controls expression of S1c25a10 gene is determined for obesity test subjects under examination, and polymorphic sites are located. The allele frequencies at the detected polymorphic sites are calculated, and polymorphisms which correlate with obesity are identified by discovering alleles which are significantly increased or decreased in the subject group. The genetic polymorphisms determined in this manner may be clinically detected in genomic DNA derived from the subject by, for example, a method of analyzing the base sequence at the polymorphic site, or utilizing differences in the physicochemical properties of DNA which vary depending on the type of base at the polymorphic site, or differences in restriction endonuclease sites, a method utilizing a detection probe suitable for detection of the polymorphic site, or a method utilizing mass spectrometry.

(E) A method of examining obesity by detecting expression or activity of protein which affects expression of S1c25a10 gene through interaction with S1c25a10 protein

Most proteins exhibit their physiological function *in vivo* by interaction with other proteins. S1c25a10 also exhibits its function with its expression controlled by the action of transcription factors, for example. A fixed correlation exists between S1c25a10 protein and expression or activity of a protein which affects expression of S1c25a10 gene by interaction with S1c25a10 protein, and the relationship is such that detection of the behavior of either allows measurement of the behavior of the other.

Here, "interaction" refers to direct or indirect action between S1c25a10 protein and a different protein, and for example, there may be mentioned action whereby physical contact between S1c25a10 protein and the different protein results in modification of amino acids, or interaction via a third protein which indirectly affects expression of S1c25a10 protein. Such proteins include, for example, proteins that exhibit their physiological function upstream or downstream from S1c25a10 protein for signal transduction via S1c25a10 protein. The method of detecting expression or activity of such a protein may be appropriately selected as a suitable means for the protein of interest, and there are no particular restrictions on the specific method.

The method of examining obesity according to the invention as explained under (A) to (E) above not only allows diagnosis of obesity or emaciation on molecular level, but also permits prognosis regarding possible future obesity or emaciation and more precise diagnosis compared to conventional diagnostic methods.

### (3) Therapeutic or preventing agents for obesity or emaciation

A correlation is seen between S1c25a10 gene expression levels and body weight. As explained above, the gene is involved in fatty acid synthesis and increase in expression of the gene leads to accelerated fatty acid synthesis. Thus, a compound that regulates the expression level of the gene to the normal level is not only useful for treatment or prevention of obesity, but can also be applied to conditions such as, for example, emaciation, diabetes, hypertension, hyperlipidemia and ischemic heart disease. Also, since inhibition of S1c25a10 gene expression inhibits fatty acid synthesis, compounds which inhibit S1c25a10 gene expression or reduce S1c25a10 protein activity function as fatty acid synthesis inhibitors. Such compounds include those selected by the method of evaluating compounds according to the invention as described above. Such compounds may be used as drugs by direct administration of the compounds to patients, or by their administration in the form of medical compositions formulated by publicly known pharmaceutical methods. For formulation, the following may be specifically mentioned as examples of pharmacologically acceptable carriers or media: sterilized water, physiological saline, vegetable oils, emulsifiers, suspending agents, surfactants, stabilizers, binders, lubricants, sweeteners, aromatics and coloring agents. As examples of methods of administering such medical compositions to patients there may be mentioned intraarterial injection, intravenous injection, subcutaneous injection, intranasal administration, transbronchial inhalation, intramuscular administration or oral administration. The amount of the medical composition administered will vary depending on the patient body weight and age and the method of administration, and a suitable dosage may be selected by a person skilled in the art.

### (4) Obesity or emaciation examination agent and examination kit

S1c25a10 protein expression levels are correlated with changes in body weight due to obesity or emaciation. Thus, antibodies against the protein can be used for detection and assay of the protein levels in test cell or test tissue to conveniently perform examination of obesity or emaciation. Here, "antibodies" may be complete antibody molecules or fragments thereof, which are able to bind S1c25a10 gene product as antigen. Such antibodies may be produced by publicly known methods, and may be either monoclonal antibodies or polyclonal antibodies. Immunological assay using such antibodies may be accomplished by a publicly known method, and specifically there may be mentioned fluorescent antibody assay and enzyme-antibody assay.

The present invention can also be implemented by producing a kit including such antibodies. The kit construction may include, in addition to an antibody, a fluorescent labeling substance for detection of the antibody, as well as a secondary antibody labeled with a radioisotope and a buffer solution to be used for antigen-antibody reaction.

By using such an examining agent for obesity or emaciation, it is possible not only to diagnose obesity on molecular level, but also to perform prognosis regarding possible future obesity or emaciation, and to achieve a more accurate diagnosis than by prior art diagnostic methods. Moreover, using an examination kit for obesity or emaciation according to the invention allows such accurate diagnosis to be carried out in a highly convenient manner.

### (5) A method of inhibiting fatty acid synthesis and a method of treating or preventing obesity

A method of inhibiting fatty acid synthesis and a method of treating or preventing obesity of the invention will now be explained. As already mentioned, the present inventors have discovered that when S1c25a10 gene expression levels are inhibited, expression levels of ACC1, a gene involved in fatty acid synthesis, and levels of malonyl-CoA, a fatty acid precursor, are reduced. Thus, by reducing expression levels of S1c25a10 gene it is possible to inhibit fatty acid synthesis, and hence synthesis of fat.

Specifically, the fatty acid synthesis inhibition is achieved in the following manner. First, a substance which reduces Slc25a10 expression is selected. The substance may be, for example, a compound which acts as an inhibitor of S1c25a10, an antibody against S1c25a10, anti-sense nucleotide or siRNA used for RNAi.

Next, the substance is introduced into an individual, tissue or cells containing S1c25a10. Specifically, if the target is an individual, the method of introduction is not particularly restricted and may be a method in which the compound, etc. is introduced by intraarterial injection, intravenous injection, subcutaneous injection, intranasal administration, transbronchial inhalation, intramuscular administration or oral administration. If the target is a tissue, the method of introduction is not particularly restricted and may be a method of injection into the tissue or introduction after admixture in a buffer. If cells are the target, the method of introduction is not particularly restricted and may be admixture in a buffer, electroporation or the like.

More specifically, RNAi can be accomplished by introduction of siRNA into cells by, for example, contacting liposome-packaged siRNA with cells added to a cell culture solution (Nature, 411, 494-498 (2001), J. Cell Sci., 114 (Pt. 24), 4557-4565 (2001), Biochem. Biophys. Res. Commun., 301(3), 804-809 (2003)). The following siRNA may be used for RNAi of S1c25a10: H1 (SEQ ID NOs: 3 and 4), H2 (SEQ ID NOs: 5 and 6), H3 (SEQ ID NOs: 7 and 8), H4 (SEQ ID NOs: 9 and 10), H5 (SEQ ID NOs: 11 and 12), H8 (SEQ ID NOs: 17 and 18), H10 (SEQ ID NOs: 21 and 22), H11 (SEQ ID NOs: 23 and 24), H12 (SEQ ID NOs: 25 and 26), M1 (SEQ ID NOs: 27 and 28), M2 (SEQ ID NOs: 29 and 30), M3 (SEQ ID NOs: 31 and 32), M5 (SEQ ID NOs: 35 and 36), M6 (SEQ ID NOs: 37 and 38), M7 (SEQ ID NOs: 39 and 40), M8 (SEQ ID NOs: 41 and 42), M9 (SEQ ID NOs: 43 and 44), M10 (SEQ ID NOs: 45 and 46), M11 (SEQ ID NOs: 47 and 48) and M12 (SEQ ID NOs: 49 and 50). RNAi may also be produced using combinations of these siRNA. Among these, H4 and M8 have particularly powerful inhibiting effects on S1c25a10 expression and are especially suited for RNAi of S1c25a10.

Fatty acid synthesis is inhibited by reducing S1c25a10 expression levels in this manner.

The fatty acid synthesis inhibition method may be applied for treatment or prevention of obesity. Specifically, treatment or prevention of obesity can be achieved by inhibiting synthesis of fatty acids, for effective suppression of lipogenesis, through *in vivo* reduction of S1c25a10 expression levels.

More specifically, treatment or prevention of obesity is achieved in the following manner. First, a substance which reduces S1c25a10 expression is selected. The substance may be, for example, a compound which acts as an inhibitor of S1c25a10, an antibody against S1c25a10, anti-sense nucleotide or siRNA used for RNAi.

Next, the substance is administered to an organism. The administration method is not particularly restricted and may be a method of intraarterial injection, intravenous injection, subcutaneous injection, intranasal administration, transbronchial inhalation, intramuscular administration or oral administration. A specific method using RNAi was explained for inhibition of fatty acid synthesis.

### (6) siRNA consisting of nucleic acids of SEQ ID NOs: 9 and 10, and S1c25a10 expression suppressors, fatty acid synthesis inhibitors and therapeutic or preventing agents for obesity which contain the same

As already explained above, siRNA consisting of nucleic acids of SEQ ID NOs: 9 and 10 powerfully inhibit S1c25a10 expression. Thus, the siRNA can be used as S1c25a10 expression suppressors, as fatty acid synthesis inhibitors, or as therapeutic or preventing agents for obesity.

### (7) siRNA consisting of nucleic acids of SEQ ID NOs: 41 and 42, and S1c25a10 expression suppressors, fatty acid synthesis inhibitors and therapeutic or preventing agents for obesity which contain the same

As already explained above, siRNA consisting of nucleic acids of SEQ ID NOs: 41 and 42 powerfully inhibit S1c25a10 expression. Thus, the siRNA can be used as S1c25a10 expression suppressors, as fatty acid synthesis inhibitors, or as therapeutic or preventing agents for obesity.

### Examples

The present invention will now be explained in greater detail by examples, with the understanding that the invention is not restricted to these examples.

### (Creation of obesity animal model)

Preparation Example 1: Mice intracerebroventicularly (i.c.v.) administered with Neuropeptide Y (NPY) Y5 agonist

A mouse model of obesity induced by administration of an NPY Y5 agonist was prepared in the following manner. Nine- to twelve-week-old male mice (C57BL/6J: Clea Japan) were raised under conditions with a room temperature of 23 ± 2 °C and a humidity of 55 ± 15 %, with one mouse in each plastic cage. The mice were raised under a 12 hour lightness/darkness cycle, with lights on at 7:00 am and lights off at 7:00 pm. The mice were also given free access to feed (CE-2 (25.4 wt% protein, 50.3 wt% carbohydrate, 4.4 wt% lipid), Clea Japan) and water.

The mice were anesthetized with 80 mg/kg sodium pentobarbital (Dynabot) and a 28-gauge sterilized brain infusion cannula (Alzet Co.) was stereotactically implanted in the right cerebral ventricle. The cannula was positioned 0.4 mm behind and 0.8 mm to the side of the bregma, and to a depth of 2 mm, and was anchored vertically with respect to the cranial bone using dental cement. A polyvinyl chloride tube was used to connect the cannula to an osmotic pump (Model #2002: Alzet Co.) filled with 10 mM phosphate buffer containing 0.05% bovine serum albumin (BSA). A solution of D-Try³⁴ NPY in 10 mM PBS (containing 0.05% BSA) (prepared for 5 µg/day) was filled into the pump, and the pump was implanted subcutaneously at the back of the mouse for continuous infusion. An antibiotic (50 mg/kg cefamedine, product of Fujisawa Pharmaceutical Co., Ltd.) was also subcutaneously injected.

The mice were divided into three groups: a normally-fed group given infusion of the solvent alone (vehicle group); an obesity-induced group given infusion of D-Try³⁴ NPY (NYP Y5 agonist) with an increased food amount (ad lib fed group); and a group given infusion of D-Try³⁴ NPY but with food restricted to the same amount as the vehicle group (pair-fed group).

### Preparation Example 2: MCH-administered mice

A mouse model of obesity induced by administration of MCH (melanin-concentrating hormone) was prepared in the following manner. Thirteen-week-old male mice (C57BL/6J: Clea Japan) were raised under conditions with a room temperature of 23 ± 2 °C and a humidity of 55 ± 15 %, with one mouse in each plastic cage. The mice were raised under a 12 hour lightness/darkness cycle, with lights on at 7:00 am and lights off at 7:00 pm. The mice were also given free access to food (CE-2 (25.4 wt% protein, 50.3 wt% carbohydrate, 4.4 wt% lipid), Clea Japan) and water. When the mice had adapted to their environment, they were given MHF (15.0 wt% protein, 52.4 wt% carbohydrate, 32.6 wt% lipid, Oriental Bioservice) as feed.

The mice were anesthetized with 80 mg/kg sodium pentobarbital (Dynabot) and a 28-gauge sterilized brain infusion cannula (Alzet Co.) was stereotactically implanted in the right cerebral ventricle. The cannula was positioned 0.4 mm behind and 0.8 mm to the side of the bregma, and to a depth of 2 mm, and was anchored vertically with respect to the cranial bone using dental cement. A polyvinyl chloride tube was used to connect the cannula to an osmotic pump (Model #2002: Alzet Co.) filled with 30% propylene glycol. The pump was implanted subcutaneously at the back of the mouse, and the mouse was subcutaneously injected with an antibiotic.

The mice were divided into three groups with equivalent average body weights: a group given infusion of the solvent alone (vehicle group); a group given infusion of MCH (ad lib fed group); and a group given infusion of MCH and pair-fed (pair-fed group). The pump was then replaced with MCH (3 µg/day) or solvent (30% propylene glycol) under ether anesthesia.

### Preparation Example 3: DIO (Diet Induced Obesity) mice

Eighteen-week-old male mice (C57BL/6J: Clea Japan) were raised under conditions with a room temperature of 23 ± 2 °C and a humidity of 55 ± 15 %, with one mouse in each plastic cage. The mice were given a high-calorie diet of MHF (18.2 wt% protein, 55.6 wt% carbohydrate, 15.5 wt% lipid) for a period of 6 months, to create an obese mouse model (DIO mice). In the examples, "established MFD" refers to mice raised with MHF feeding until body weight no longer increased.

Also created were DIO mice (HFD), which were the same mice given a high-calorie diet of HFD (20.8 wt% protein, 38.59 wt% carbohydrate, 32.88 wt% lipid) containing more fat than MHF.

### Preparation Example 4: Dietary-restricted mice

Mice (C57BL/6N, 17-week-old) were raised each separately in different cages. The feed given was ordinary feed (CA-1, Clea Japan). Dietary restriction was carried out according to the following schedule. Specifically, the feed (CA-1) was supplied for 3 hours each day (10:00-13:00), while water was made freely available. The feed weight was measured before and after the feeding time, and the difference was calculated as the ingested weight. The body weights and appearances were observed during the period of dietary restriction. Mice believed to have failed the conditions (mice which exhibited an excessive body weight decrease (for example, about a 20% decrease) in a short time) were not used for the experiment. After 7 days of raising the mice under these conditions, the white adipocytes were extracted.

### Examples 1-5 and Comparative Examples 1 and 2: S1c25a10 expression in white adipocytes

The mouse models prepared in Preparation Examples 1-4 were used for measurement of S1c25a10 expression in white adipocytes (WAT). The expression levels were measured by treating RNA extracted from white adipocytes from each mouse model using a mouse U74A chip (Affymetrix) and a mouse 25K1.8 chip (Rossetta).

Table 1 shows S1c25a10 gene expression levels for DIO mice (DIO), D-Try³⁴ NPY-administered mice (NPY(FF)), D-Try³⁴ NPY pair feeding mice (NPY(PF)), MCH-administered mice (MCH(FF)), MCH pair feeding mice (MCH(PF)), dietary-restricted mice (Fasting) and NPY Y5 agonist-administered mice (Y5ant), where the S1c25a10 expression WAT of non-treated C57BL/6N mice was defmed as 1.

As shown in Table 1, S1c25a10 gene expression tended to increase in the obese mouse models, while the expression decreased in the dietary-restricted mice and the NPY Y5 agonist-administered mice. Thus, a clear correlation was established between S1c25a10 expression level and body weight.

**Table 1**

| | Obesity model | S1c25a10 expression |
|---|---|---|
| Example 1 | DIO mice | 1.9 |
| Example 2 | NPY(PF) | 1.9 |
| Example 3 | NPY(FF) | 3.0 |
| Example 4 | MCH(PF) | 2.4 |
| Example 5 | MCH(FF) | 1.7 |
| Comp. Example 1 | Fasting | 0.2 |
| Comp. Example 2 | Y5 antagonist | 0.77 |

### Example 6: Relationship between S1c25a10 and mitochondrial proton gradient

UCP, which has high homology with S1c25a10, mediates thermogenesis by varying the membrane potential in mitochondria. S1c25a10 was therefore also examined with regard to mitochondrial proton gradient.

First, HEK293 cells were seeded in a 6-well plate at a density of 2 x 10⁶ cells/well. After 24 hours, S1c25a10 gene cloned in pcDNA 3.1 vector was transfected into the cells using Lipofectamine 2000. As a negative control there were used cells having only the vector transfected, and as positive controls there were used cells having mouse UCP1 transfected and cells having human UCP3 transfected.

After 48 hours from transfection of the gene, the mitochondrial gradient sensitivity-indicating agent DiOC6 (3-3'-dihexyloxacarbocyanine iodide) was used for staining. The staining was performed by treating the cells with 0.3 µM DiOC6 for 20 minutes followed by rinsing twice with PBS. DiOC6 binds to mitochondria in greater amount as the proton gradient increases, emitting strong fluorescence. On the other hand, it binds to mitochondria in lower amount as the proton gradient decreases, resulting in weaker fluorescent intensity.

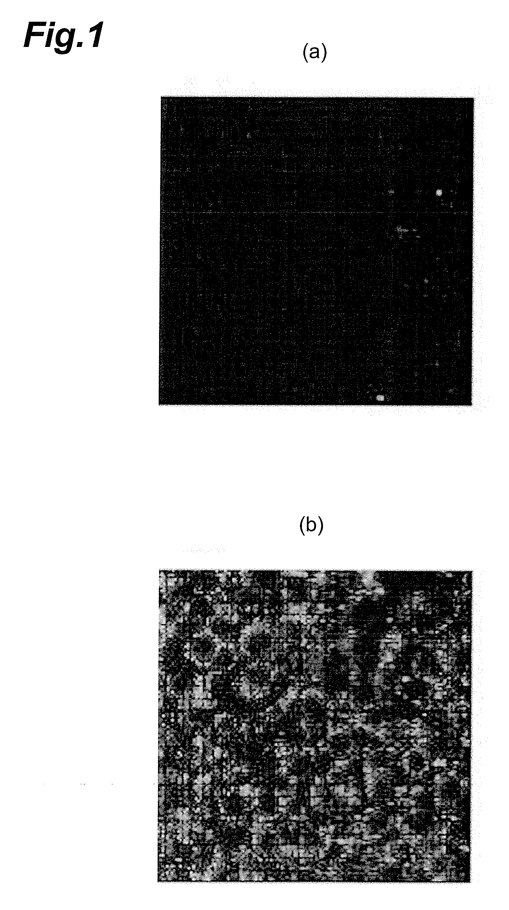

Fig. 1(a) is a confocal micrograph showing DiOC6-stained HEK293 cells treated with CCCP (carbonyl cyanide m-chlorophenylhydrazone) which uncouples the intracellular mitochondrial proton gradient, and Fig. 1(b) is a photograph of HEK293 cells without CCCP treatment. With the untreated HEK293 cells, the intracellular mitochondria are clearly stained by outlining dots. In the CCCP-treated cells, however, the fluorescent intensity is significantly reduced. It was thus confirmed that the mitochondria had been stained in a proton gradient-specific manner.

Quantitation of the fluorescent intensity of DiOC6, i.e. the mitochondrial proton gradient, was accomplished by the following two methods.

### (1) Analysis by flow cytometer

After rinsing the DiOC6-stained cells with phosphate buffered saline (PBS), a flow cytometer (Epics Elite Flow Cytometer, product of Beckman Coulter) (argon laser: 488 nm, band pass filter: 522 nm) was used for measurement of the fluorescent intensity.

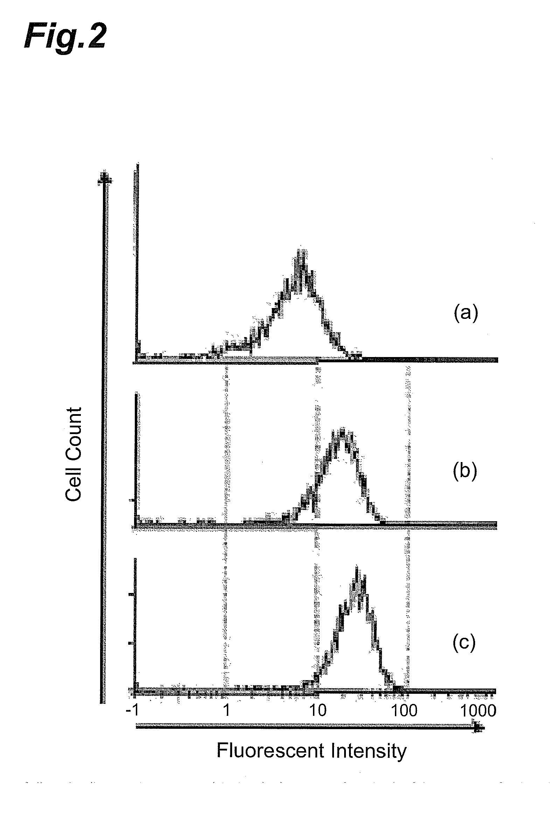

The flow cytometer analysis results are shown in Fig. 2. Here, (a) represents HEK293 cells treated with CCCP, (b) represents non-treated HEK293 cells and (c) represents S1c25a10-transfected HEK293 cells. As shown in Fig. 2(a) to (c), the histogram representing the fluorescent intensity of the S1c25a10-transfected HEK293 cells is shifted (increase in fluorescence intensity) compared to the control, thus confirming an increase in proton gradient. Table 2 shows the mean values for the fluorescent intensity of each sample.

**Table 2**

| | Sample name | Mean fluorescent intensity |
|---|---|---|
| Fig. 2(a) | CCCP-treated | 5.4 |
| Fig. 2(b) | Slc25a10 gene non-transfected | 16.8 |
| Fig. 2(c) | Slc25a10 gene-transfected | 25.9 |

### (2) Analysis by fluorometer

After rinsing DiOC6-stained cells with PBS, they were lysed and centrifuged to prepare a cell extract. The cell extract was analyzed using a fluorometer (Cytofluor Series 4000 Fluorescent Multi Well Plate Reader; Perseptive Biosystems) (Excitation: 485 nm, Emission: 520 nm).

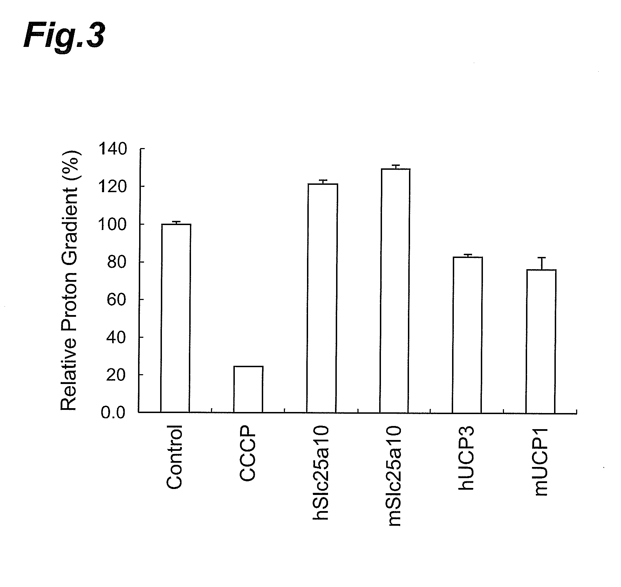

The relative value for each sample was determined with respect to 100 as the fluorescent intensity of control cells with only the vector transfected, and as shown in Fig. 3, a decrease in proton gradient of about 20% was observed in the mouse UCP1- and human UCP3-transfected cells, while an increase in proton gradient of about 20% was observed in the mouse or human S1c25a10 gene-transfected cells. These results confirmed that the activity of S1c25a10 on proton gradient is opposite to the effect of UCP, and that the strength of activity is approximately equivalent to that of UCP.

### Example 7: S1c25a10 gene expression

S1c25a10 gene expression tissue analysis was conducted using a Northern analysis membrane (BioChain Corp., Frontech Corp.) onto which mRNA from adipose tissue, skeletal muscle, spleen, lung, kidney, brain, heart, testes and liver had been transferred. The probe used was full-length cDNA of mouse S1c25a10 labeled with ³²P, and hybridization was carried out using QuickHYB (Stratagene) as a buffer.

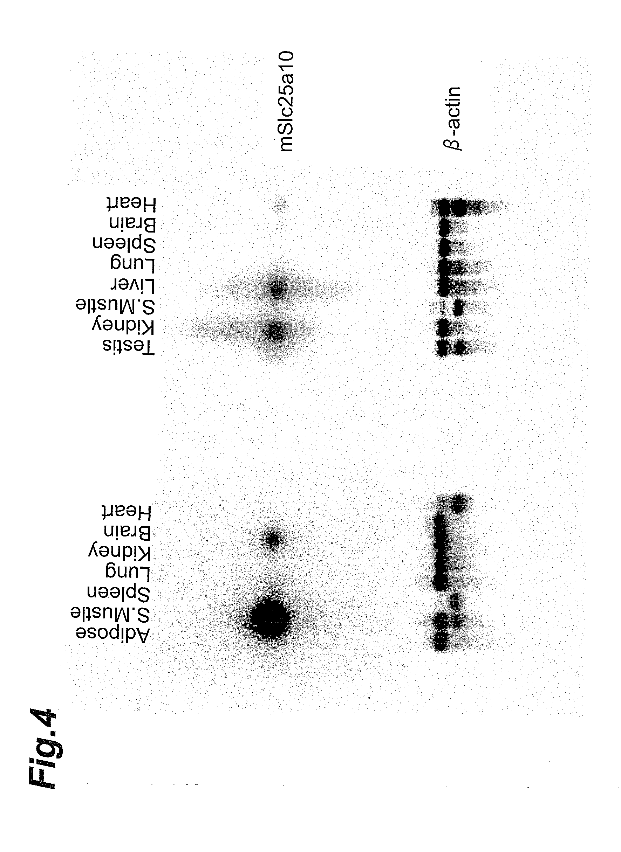

As shown in Fig. 4, S1c25a10 gene is very highly expressed in adipose tissue, and also slightly expressed in kidney and liver.

### Example 8: Behavior of ACC1 under suppression of S1c25a10 expression

### (1) Role of S1c25a10 in fatty acid synthesis

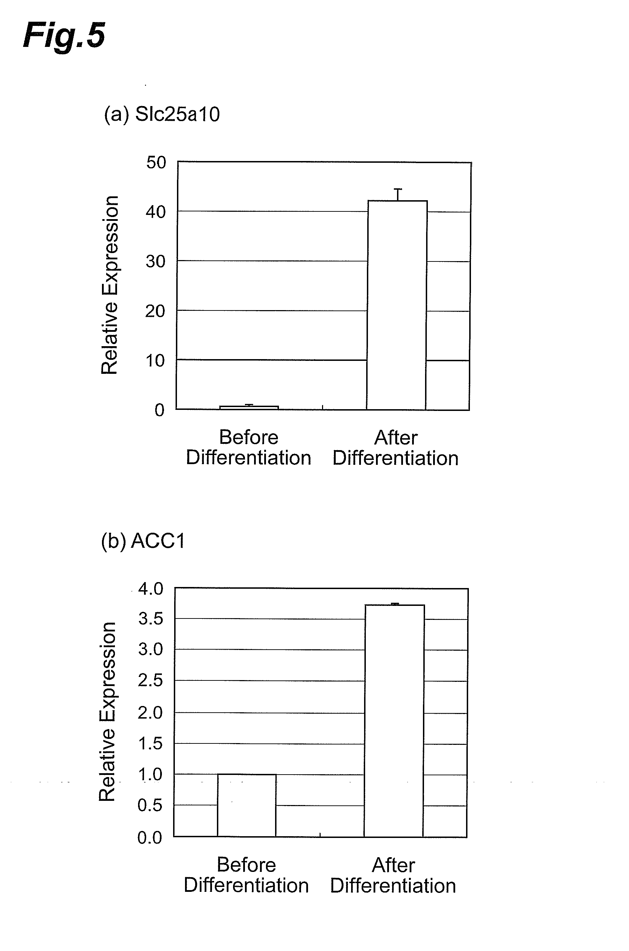

Using 3T3-L1 cells which have the potential to differentiate into adipocytes, it was confirmed whether or not a correlation exists between S1c25a10 gene and ACC1 gene expression before and after differentiation. The 3T3-L1 cells were seeded in a 6-well plate, and upon reaching confluency after 2 days, they were differentiated into adipocytes in differentiation-inducing medium containing insulin, dexamethasone and IBMX (3-isobutyl-1-methylxanthine). At 2 and 4 days after differentiation, S1c25a10 expression was suppressed for 3 hours with siRNA (M8). The correlation was examined on the 8th day after differentiation. As shown in Fig. 5(a) and (b), increases in expression were found for both S1c25a10 gene and the ACC1 gene upon differentiation of 3T3-L1 to adipocytes. Since increase in ACC1 expression is an index of enhanced fatty acid synthesis, this confirmed a close relationship between fatty acid synthesis enhancement and S1c25a10 expression.

### (2) siRNA transfection and quantitative RT-PCR

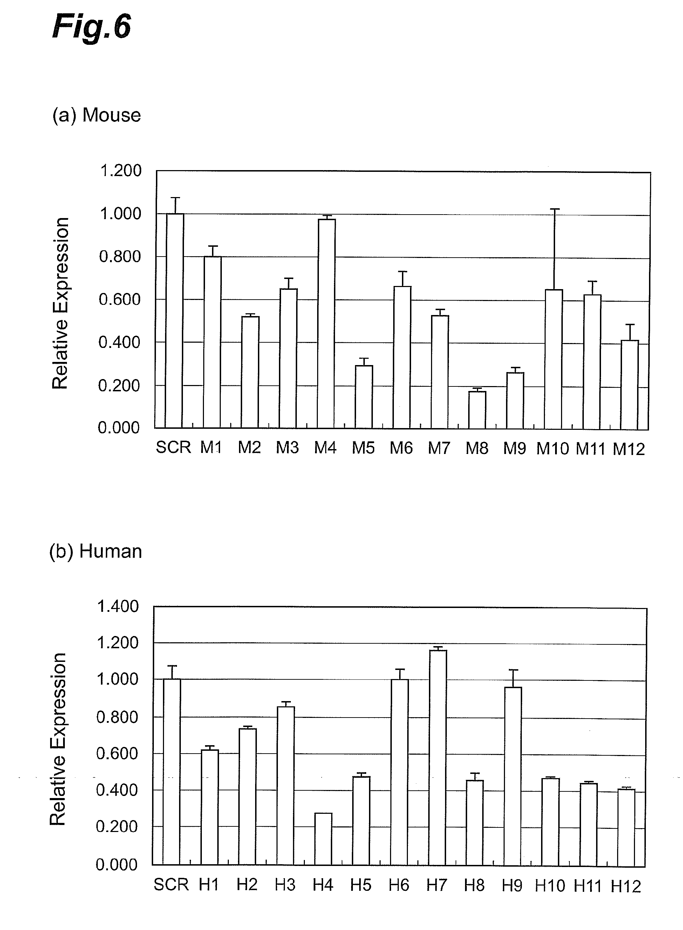

First, a silencer siRNA construction kit (Ambion Inc.) was used to synthesize siRNA. Next, in order to determine the optimum sequences for human or mouse S1c25a10, twelve different sequences were examined based on suppression of S1c25a10 expression. Fig. 6(a) and (b) show the relative expression levels of S1c25a10 gene in each of the siRNA-transfected cells. Based on these results, H4, H10 and M8 were used as the siRNA for the subsequent experiment.

The sequence of each siRNA is shown below. The "Position" indicates the corresponding nucleic acid position of the human S1c25a10 gene (NM_012140) for each siRNA.

H1 (Position 186)

Sense: AACTGCGTCTGCAGATGCACCCCTGTCTC (SEQ ID NO: 3)
Antisense: AAGGTGCATCTGCAGACGCAGCCTGTCTC (SEQ ID NO: 4)

H2 (Position 465)
Sense: AAGTCGTTCTGCATCCTGACGCCTGTCTC (SEQ ID NO: 5)
Antisense: AACGTCAGGATGCAGAACGACCCTGTCTC (SEQ ID NO: 6)

H3 (Position 513)
Sense: AAATCCAGCGCATGGGCGTAGCCTGTCTC (SEQ ID NO: 7)
Antisense: AACTACGCCCATGCGCTGGATCCTGTCTC (SEQ ID NO: 8)

H4 (Position 556)
Sense: AAACAGTCTCCTGAGACCCTCCCTGTCTC (SEQ ID NO: 9)
Antisense: AAGAGGGTCTCAGGAGACTGTCCTGTCTC (SEQ ID NO: 10)

H5 (Position 651)
Sense: AAGGTGCTAAGGACCAGCTGCCCTGTCTC (SEQ ID NO: 11)
Antisense: AGCAGCTGGTCCTTAGCACCCCTGTCTC (SEQ ID NO: 12)

H6 (Position 780)
Sense: AACTGATACTCCCCCTTGGAGCCTGTCTC (SEQ ID NO: 13)
Antisense: AACTCCAAGGGGGAGTATCAGCCTGTCTC (SEQ ID NO: 14)

H7 (Position 945)
Sense: AAGGCTGGTCAGGATGGCACTCCTGTCTC (SEQ ID NO: 15)
Antisense: AAAGTGCCATCCTGACCAGCCCCTGTCTC (SEQ ID NO: 16)

H8 (Position 1010)
Sense: AAGTGCTGGGCTTGGGACTCTCCTGTCTC (SEQ ID NO: 17)
Antisense: AAAGAGTCCCAAGCCCAGCACCCTGTCTC (SEQ ID NO: 18)

H9 (Position 1315)
Sense: AAGTGCTGGAAGATGCTGCTCCTGTCTC (SEQ ID NO: 19)
Antisense: AAAGTGCTGGAAGATGCTGCTCCTGTCTC (SEQ ID NO: 20)

H10 (Position 1426)
Sense: AAGAGGACATGGAAGGTCTGGCCTGTCTC (SEQ ID NO: 21)
Antisense: AACCAGACCTTCCATGTCCTCCCTGTCTC (SEQ ID NO: 22)

H11 (Position 1634)
Sense: AAGCTGGTGAGTGGAGAGGCTCCTGTCTC (SEQ ID NO: 23)
Antisense: AAAGCCTCTCCACTCACCAGCCCTGTCTC (SEQ ID NO: 24)

H12 (Position 1870)
Sense: AAAGCTCCCGGCATTTATTGACCTGTCTC (SEQ ID NO: 25)
Antisense: AATCAATAAATGCCGGGAGCTCCTGTCTC (SEQ ID NO: 26)

The nucleotide sequences of siRNA corresponding to the mouse S1c25a10 gene (NM_013770)

M1 (Position 209)
Sense: AATTGGGTCTGCAAATGCACCCCTGTCTC (SEQ ID NO: 27)
Antisense: AAGGTGCATTTGCAGACCCAACCTGTCTC (SEQ ID NO: 28)

M2 (Position 358)
Sense: AATCCCGCATGGTCTCGTAGACCTGTCTC (SEQ ID NO: 29)
Antisense: AATCTACGAGACCATGCGGGACCTGTCTC (SEQ ID NO: 30)

M3 (Position 488)
Sense: AAGTCGTTCTGCATCCTGACACCTGTCTC (SEQ ID NO: 31)
Antisense: AATGTCAGGATGCAGAACGACCCTGTCTC (SEQ ID NO: 32)

M4 (Position 536)
Sense: AAATCCAGGGCATGAGAGTAGCCTGTCTC (SEQ ID NO: 33)
Antisense: AACTACTCTCATGCCCTGGATCCTGTCTC (SEQ ID NO: 34)

M5 (Position 674)
Sense: AAAGTGCTGAGGACCAGTTGCCCTGTCTC (SEQ ID NO: 35)
Antisense: AAGCAACTGGTCCTCAGCACTCCTGTCTC (SEQ ID NO: 36)

M6 (Position 788)
Sense: AAGGAGTTCATCAGGCGAGTCCCTGTCTC (SEQ ID NO: 37)
Antisense: AAGACTCGCCTGATGAACTCCCCTGTCTC (SEQ ID NO: 38)

M7 (Position 968)
Sense: AAATGTCAGGTGGTTGGCACTCCTGTCTC (SEQ ID NO: 39)
Antisense: AAAGTGCCAACCACCTGACATCCTGTCTC (SEQ ID NO: 40)

M8 (Position 1159)
Sense: AAGTGGCACCTCTGCCCTACTCCTGTCTC (SEQ ID NO: 41)
Antisense: AAAGTAGGGCAGAGGTGCCACCCTGTCTC (SEQ ID NO: 42)

M9 (Position 1312)
Sense: AAAGCAGGAAACGAACTCGGCCCTGTCTC (SEQ ID NO: 43)
Antisense: AAGCCGAGTTCGTTTCCTGCTCCTGTCTC (SEQ ID NO: 44)

M10 (Position 1481)
Sense: AACTCTCCTGAAGGCACTACCCCTGTCTC (SEQ ID NO: 45)
Antisense: AAGGTAGTGCCTTCAGGAGAGCCTGTCTC (SEQ ID NO: 46)

M11 (Position 1661)
Sense: AAGTGTGAGGGACACAGACAGCCTGTCTC (SEQ ID NO: 47)
Antisense: AACTGTCTGTGTCCCTCACACCCTGTCTC (SEQ ID NO: 48)

M12 (Position 1827)
Sense: AATTGAGGGAAAACAGGCTGCCCTGTCTC (SEQ ID NO: 49)
Antisense: AAGCAGCCTGTTTTCCCTCAACCTGTCTC (SEQ ID NO: 50)

Next, siRNA-transfected cells were prepared. For siRNA transfection, the cells were seeded on a 6-well plate at a density of 2 x 10⁴ cells/well. After 24 hours, the siRNA (H4 or H 10) was transfected into the cells using Oligofectamine (Invitrogen). On the 2nd day after transfection, the cells were harvested and RNA was prepared using RNeasy (Qiagen). cDNA was prepared from the RNA by quantitative RT-PCR (Applied Biosystems), for quantitation of the S1c25a10 and ACC1 expression levels.

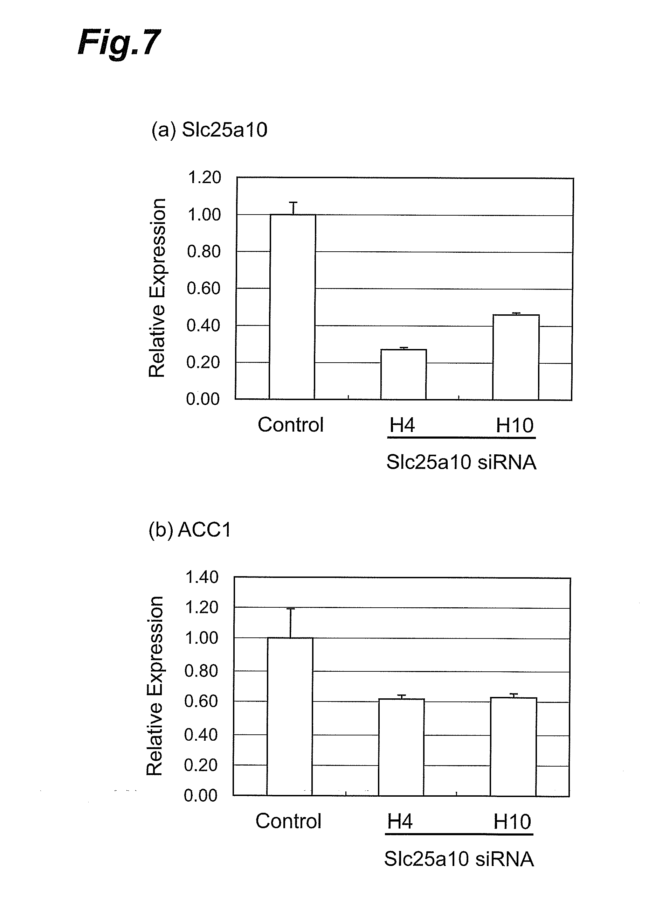

As shown in Fig. 7(a) and (b), it was confirmed that suppressing expression of S1c25a10 gene by RNAi also suppressed ACC1 gene expression in a proportional manner.

### Example 9: Behavior of ACC1 under forced expression of S1c25a10

Expression of S1c25a10 gene was then forced for increased expression in HEK293 cells, and the behavior of ACC1 expression was investigated.

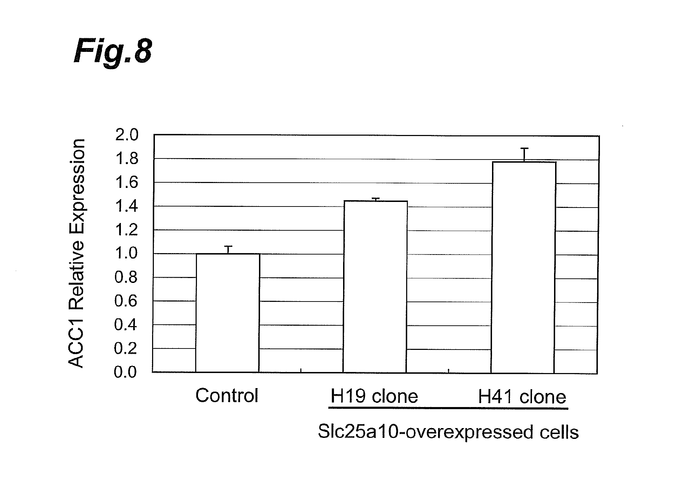

First, an S1c25a10 expression vector was prepared by cloning of S1c25a10 gene in pcDNA3.1 (Invitrogen). The expression vector was transfected into HEK293 cells and cell lines with stable S1c25a10 expression (H19 clone and H41 clone) were established. RNA was prepared from each cell line (10⁶ cells), and the S1c25a10 and ACC1 expression levels were measured by quantitative RT-PCR of the prepared RNA. As shown in Fig. 8, it was confirmed that forced expression of S1c25a10 gene also increased expression of the ACC1 gene, compared to the control.

### Example 10: Behavior of malonyl-CoA with Slc25a10 expression

HepG2 cells cultured at a density of 10⁷ cells/dish were trypsin-treated and centrifuged, and the cells were collected. Trichloroacetic acid (10%; 800 µL) was added to the obtained cell pellet, and after centrifugation at 3000 rpm for 10 minutes, the solubilized fraction was extracted. For partial purification of malonyl-CoA, the extract was purified by reverse-phase chromatography (Sep-Pak C18). The obtained eluate was dried and then dissolved in 100 µL of water. A sample containing malonyl-CoA (50 µL) was reacted with fatty acid synthase and radioactive-labeled acetyl-CoA for fatty acid synthesis. The fatty acids were extracted with petroleum ether and the radioactivity of the radioactive-labeled fatty acids was measured with a scintillation counter.

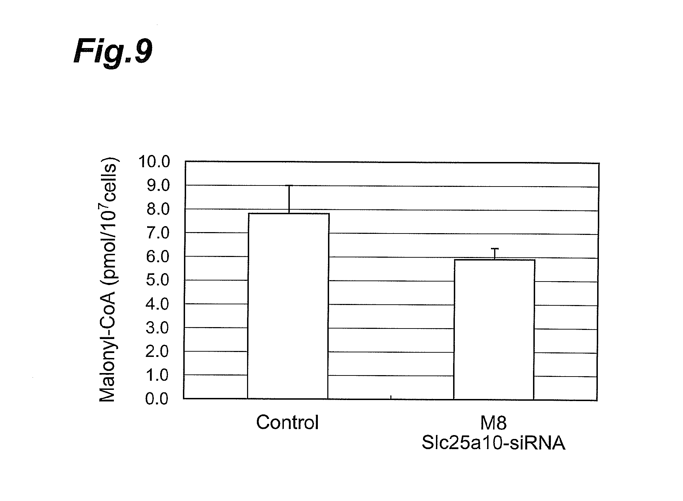

As shown in Fig. 9, it was confirmed that suppressing expression of S1c25a10 gene reduced malonyl-CoA production. Also, since fatty acid production normally occurs with behavior proportional to malonyl-CoA, this suggested a similar relationship between S1c25a10 gene expression and fatty acid production.

### Example 11: Identification of fat accumulation

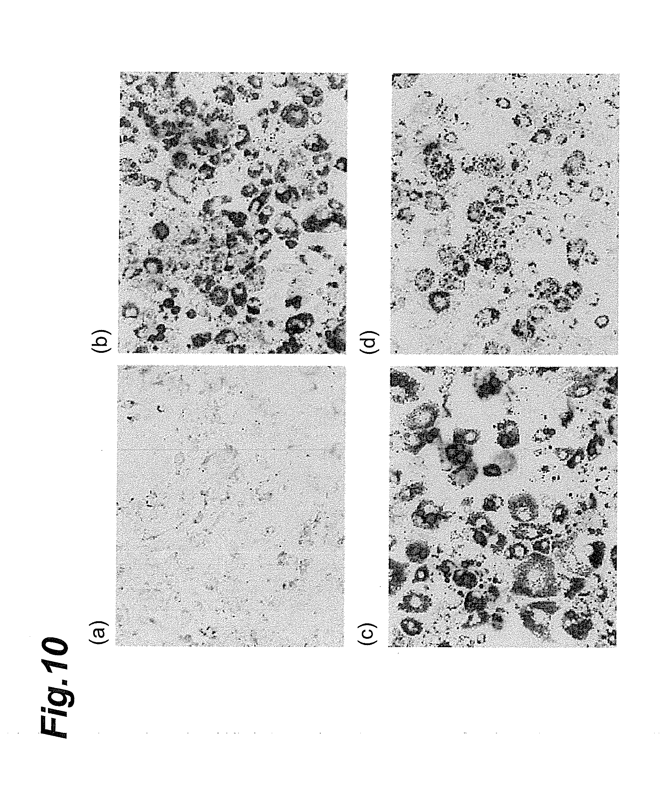

3T3-L1 cells were used for analysis of fat accumulation.
3T3-L1 cells were seeded in a 6-well plate, and upon reaching confluency after 2 days, they were differentiated into adipocytes in differentiation-inducing medium containing insulin, dexamethasone and IBMX (3-isobutyl-1-methylxanthine). At 2 and 4 days after differentiation, S1c25a10 expression was suppressed for 3 hours with siRNA (M8). On the 8th day after differentiation, the accumulated fat was stained with 0.175% Oil Red O and rinsed with PBS. Fig. 10 is a micrograph of 3T3-L1 cells differentiated to adipocytes.

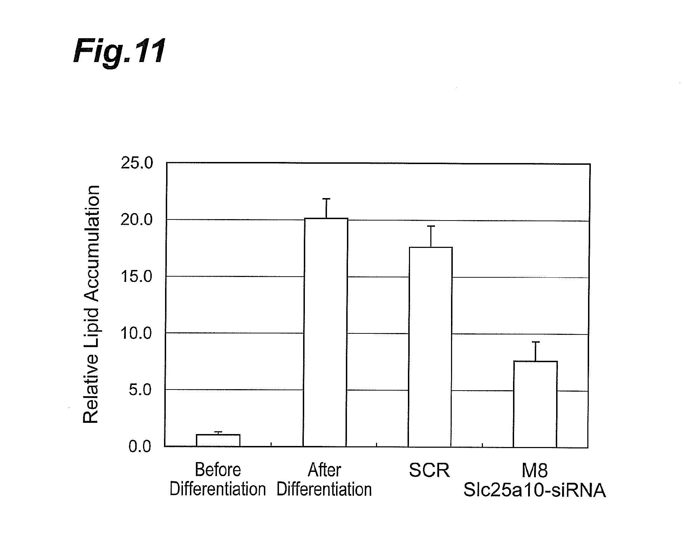

For quantitation of fat accumulation, the stained fat cells were treated with 1 mL of propanol for elution of the accumulated fat. The results of quantitation of fat accumulation in each of the cells is shown in Fig. 11. As clearly seen by the results shown in Figs. 10 and 11, suppression of S1c25a10 expression by RNAi resulted in reduced accumulation of fat.

### Industrial Applicability

As explained above, the compound evaluation method and examination method according to the invention allows evaluation of compounds, including screening of therapeutic agents, for obesity. In addition, it is possible to provide an examination method for obesity which permits judgment to be made on molecular level. Thus, anti-obesity drug development and clinical diagnosis of obesity can be provided, in order to achieve new drugs and diagnostic tools in the field of medicine for this lifestyle disease.

According to the present invention, it is also possible to provide a method for treatment and prevention of metabolic disorders, circulatory diseases, central nervous system disorders and the like using substances (for example, siRNA, low molecular compounds, proteins, antibodies and the like) having activity which inhibits long chain fatty acid elongase activity, as well as therapeutic and prophylactic agents comprising such substances. As examples of metabolic disorders there may be mentioned obesity, diabetes, hormone secretion imbalances, hyperlipidemia, gout and fatty liver. As examples of circulatory diseases there may be mentioned angina, acute and congestive heart failure, myocardial infarction, coronary sclerosis, hypertension, kidney disease and electrolyte imbalances. As an example of a nervous system disorder there may be mentioned bulimia.

## Claims

1. A method of evaluating compounds which are effective for treatment or prevention of obesity comprising
a step in which a test compound is administered to or contacted with a test animal or a test cell, and
a step of detecting change in the expression level of S1c25a10 gene or a gene which is functionally equivalent to said gene, in said test animal or test cell.

2. A method of evaluating compounds which are effective for treatment or prevention of obesity comprising
a step in which a test compound is administered to or contacted with a test animal or a test cell possessing a fusion gene comprising the expression regulatory region of S1c25a10 gene and a reporter gene, and
a step of detecting change in the expression level of said reporter gene in said test animal or test cell.

3. A method of evaluating compounds according to claim 1 or 2, wherein said change in expression level is a reduction in the expression level.

4. A method of evaluating compounds according to any one of claims 1 to 3, further comprising a step of detecting change in at least one selected from ACC1 expression, malonyl-CoA abundance and fatty acid abundance.

5. A method of evaluating compounds which are effective for treatment or prevention of obesity comprising
a step in which a test compound is administered to or contacted with a test animal or a test cell, and
a step in which it is confirmed whether or not said test compound exhibits an effect on the activity of S1c25a10 protein.

6. A method of evaluating compounds which are effective for treatment or prevention of obesity comprising
a step in which a test compound is contacted with S1c25a10 protein, and
a step in which it is confirmed whether or not said test compound exhibits an effect on the activity of said protein.

7. An agent for treatment or prevention of obesity containing as an active ingredient a compound obtained by an evaluation method according to any one of claims 1 to 6.

8. A method of inhibiting fatty acid synthesis by lowering the expression level of S1c25a10 gene.

9. A method of inhibiting fatty acid synthesis by lowering the expression level of S1c25a10 gene by RNAi.

10. A method of inhibiting fatty acid synthesis according to claim 9, wherein said RNAi is accomplished by using one or more siRNA selected from the group consisting of siRNA consisting of the nucleic acids of SEQ ID NOs: 3 and 4, siRNA consisting of the nucleic acids of SEQ ID NOs: 5 and 6, siRNA consisting of the nucleic acids of SEQ ID NOs: 7 and 8, siRNA consisting of the nucleic acids of SEQ ID NOs: 9 and 10, siRNA consisting of the nucleic acids of SEQ ID NOs: 11 and 12, siRNA consisting of the nucleic acids of SEQ ID NOs: 17 and 18, siRNA consisting of the nucleic acids of SEQ ID NOs: 21 and 22, siRNA consisting of the nucleic acids of SEQ ID NOs: 23 and 24, siRNA consisting of the nucleic acids of SEQ ID NOs: 25 and 26, siRNA consisting of the nucleic acids of SEQ ID NOs: 27 and 28, siRNA consisting of the nucleic acids of SEQ ID NOs: 29 and 30, siRNA consisting of the nucleic acids of SEQ ID NOs: 31 and 32, siRNA consisting of the nucleic acids of SEQ ID NOs: 35 and 36, siRNA consisting of the nucleic acids of SEQ ID NOs: 37 and 38, siRNA consisting of the nucleic acids of SEQ ID NOs: 39 and 40, siRNA consisting of the nucleic acids of SEQ ID NOs: 41 and 42, siRNA consisting of the nucleic acids of SEQ ID NOs: 43 and 44, siRNA consisting of the nucleic acids of SEQ ID NOs: 45 and 46, siRNA consisting of the nucleic acids of SEQ ID NOs: 47 and 48, and siRNA consisting of the nucleic acids of SEQ ID NOs: 49 and 50.

11. A method of inhibiting fatty acid synthesis according to claim 9, wherein said RNAi is accomplished using siRNA consisting of the nucleic acids of SEQ ID NOs: 9 and 10.

12. A method of inhibiting fatty acid synthesis according to claim 9, wherein said RNAi is accomplished using siRNA consisting of the nucleic acids of SEQ ID NOs: 41 and 42.

13. A method of treating or preventing obesity comprising a step of lowering S1c25a10 gene expression level.

14. A method of treating or preventing obesity by lowering S1c25a10 gene expression level using RNAi.

15. A method of treating or preventing obesity according to claim 14, wherein said RNAi is accomplished by using one or more siRNA selected from the group consisting of siRNA consisting of the nucleic acids of SEQ ID NOs: 3 and 4, siRNA consisting of the nucleic acids of SEQ ID NOs: 5 and 6, siRNA consisting of the nucleic acids of SEQ ID NOs: 7 and 8, siRNA consisting of the nucleic acids of SEQ ID NOs: 9 and 10, siRNA consisting of the nucleic acids of SEQ ID NOs: 11 and 12, siRNA consisting of the nucleic acids of SEQ ID NOs: 17 and 18, siRNA consisting of the nucleic acids of SEQ ID NOs: 21 and 22, siRNA consisting of the nucleic acids of SEQ ID NOs: 23 and 24, siRNA consisting of the nucleic acids of SEQ ID NOs: 25 and 26, siRNA consisting of the nucleic acids of SEQ ID NOs: 27 and 28, siRNA consisting of the nucleic acids of SEQ ID NOs: 29 and 30, siRNA consisting of the nucleic acids of SEQ ID NOs: 31 and 32, siRNA consisting of the nucleic acids of SEQ ID NOs: 35 and 36, siRNA consisting of the nucleic acids of SEQ ID NOs: 37 and 38, siRNA consisting of the nucleic acids of SEQ ID NOs: 39 and 40, siRNA consisting of the nucleic acids of SEQ ID NOs: 41 and 42, siRNA consisting of the nucleic acids of SEQ ID NOs: 43 and 44, siRNA consisting of the nucleic acids of SEQ ID NOs: 45 and 46, siRNA consisting of the nucleic acids of SEQ ID NOs: 47 and 48, and siRNA consisting of the nucleic acids of SEQ ID NOs: 49 and 50.

16. A method of treating or preventing obesity according to claim 14, wherein said RNAi is accomplished using siRNA consisting of the nucleic acids of SEQ ID NOs: 9 and 10.

17. A method of treating or preventing obesity according to claim 14, wherein said RNAi is accomplished using siRNA consisting of the nucleic acids of SEQ ID NOs: 41 and 42.

18. A method of examining obesity by assaying expression level and change in expression level of S1c25a10 gene in a test tissue or a test cell.

19. A method of examining obesity by assaying expression levels and change in expression level of S1c25a10 protein in a test tissue or a test cell.

20. A method of examining obesity by assaying change in the amount of a substance involved in fatty acid synthesis resulting from change in expression level of S1c25a10 gene or activity of S1c25a10 protein in a test tissue or a test cell.

21. A method of examining obesity by detecting a polymorphism in S1c25a10 gene in a test tissue or a test cell.

22. A method of examining obesity by detecting expression or activity of a protein which affects expression of S1c25a10 gene through interaction with S1c25a10 protein.

23. siRNA consisting of the nucleic acids of SEQ ID NOs: 9 and 10.

24. An S1c25a10 expression inhibiting agent comprising siRNA according to claim 23.

25. A fatty acid synthesis inhibiting agent comprising siRNA according to claim 23.

26. A therapeutic or preventing agent for obesity comprising siRNA according to claim 23.

27. siRNA comprising of the nucleic acids of SEQ ID NOs: 41 and 42.

28. An S1c25a10 expression inhibiting agent comprising siRNA according to claim 27.

29. A fatty acid synthesis inhibiting agent comprising siRNA according to claim 27.

30. A therapeutic or preventing agent for obesity comprising siRNA according to claim 27.
